# EUROPEAN PATENT APPLICATION

(11) **EP 0 722 702 A1**
(43) Date of publication of application: **24.07.1996**
(21) Application number: 96200126.9
(22) Date of filing: 19.01.1996
(51) Int. Cl.: A61F 2/06, A61M 25/01

(54) **Expandable carrier balloon for a stent assembly**

(30) Priority: 19.01.1995 NL 9500095
(71) Applicant: CORDIS EUROPA N.V., NL-9301 LJ Roden (NL)
(72) Inventor: The designation of the inventor has not yet been filed
(74) Representative: 't Jong, Bastiaan Jacobus

(57) **Abstract**

Expandable carrier balloon for a stent assembly, comprising an expandable sleeve enclosing the balloon and filled with a material setting under the influence of radiation, and a radiation conducting optical fiber extending to within the balloon, wherein that at least the front end of the balloon carries material impervious for radiation. In this way it is prevented that the radiation (UV- or laser radiation) reaches the blood and/or body tissue of the treated patient.

## Description

The invention relates to an expandable carrier balloon for a stent assembly comprising an expandable sleeve enclosing the balloon and filled with material setting under the influence of radiation, and a radiation conducting optical fiber extending to within the balloon.

Such a carrier balloon and the stent assembly associated therewith are described in EP-A-0 521 573 and EP-A-0 617 930, both in the name of applicant.

When using this stent assembly known as such, the still rolled up balloon connected to the distal end of a suitable catheter and having the also rolled up stent connected thereto is brought to the desired place in a body vessel by manipulation of the catheter, whereafter the stent is brought in the desired form by expanding the balloon by supplying medium under pressure through the catheter, and the setting material is set under influence of radiation (UV- or laser radiation) supplied through the optical fiber extending through the catheter and coming out the end of this fiber.

The radiation coming out of the optical fiber is for the major part absorbed by the setting material in the stent and in such way cannot reach the tissue or blood of the treated patient. However, from the front end of the balloon still radiation can leave that reaches the tissue or blood.

The invention is based on the recognition that it is very desirable that also this radiation is blocked. In view of this according to the invention it is proposed that at least the front end of the balloon carries material which is impervious for radiation.

In this way it is prevented with certainty that any tissue or blood of the patient is exposed to radiation.

Preferably the material impervious for radiation is radiation reflecting.

Preferably the material impervious for radiation is located within the balloon.

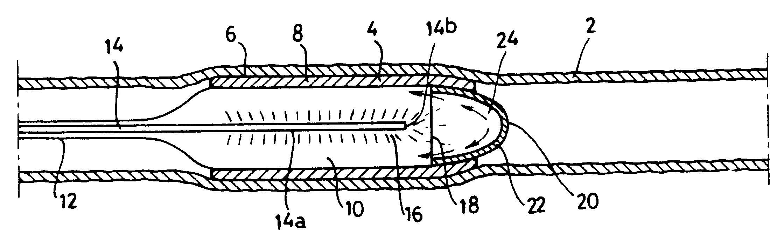

The invention is illustrated by means of the drawing.

The only figure thereof shows in longitudinal section a blood vessel with a stent assembly according to the invention arranged therein.

In the figure the reference numeral 2 indicates a blood vessel in which the elongated stent 4 is arranged. This stent is of the kind as described in EP-A-0 521 593 and EP-A-0 617 930; it comprises a double-walled sleeve 6 of which the space between the walls is filled with setting material 8. The stent is arranged in the correct position and expanded by means of an expandable balloon 10 which is arranged at the distal end of a catheter 12. The catheter 12 receives an optical fiber 14 the distal end 14a of which extends up into the balloon; the proximal end of the fiber 14 cooperates with a source of radiation (e.g. a source of UV radiation or a laser, not shown) and the radiation leaving the distal end 14a is destined to start and accelerate the setting process of the setting material in the stent.

The radiation 16 leaving the fiber at its side surface is absorbed by the setting material in the stent and therefore cannot endanger the patient. So as to prevent that radiation 18 leaving the front end 14b of the optical fiber illuminates tissue or blood of the patient, according to the invention a shielding coating 22 impervious for radiation is applied to the front end 20 of the balloon 10. Preferably this coating is reflecting for radiation, so that radiation impinging thereon, schematically indicated with 24, is reflected back to the setting material in the stent.

## Claims

1. Expandable carrier balloon for a stent assembly, comprising an expandable sleeve enclosing the balloon and filled with material setting under the influence of radiation, and a radiation conducting optical fiber extending to within the balloon, characterized in that at least the front end of the balloon carries a material impervious to radiation.

2. Balloon according to claim 1, characterized in that the material impervious to radiation is radiation reflecting.

3. Balloon according to claim 1-2, characterized in that the material impervious to radiation is located within the balloon.
